(19) **Europäisches Patentamt**

**European Patent Office**

**Office européen des brevets**

(11) **EP 1 502 546 A1**

(12) **EUROPEAN PATENT APPLICATION**

(43) Date of publication:
**02.02.2005 Bulletin 2005/05**

(51) Int Cl.⁷: **A61B 5/08**, A61B 5/0205,
A61B 5/11

(21) Application number: **04077060.4**

(22) Date of filing: **16.07.2004**

(84) Designated Contracting States:
**AT BE BG CH CY CZ DE DK EE ES FI FR GB GR
HU IE IT LI LU MC NL PL PT RO SE SI SK TR**
Designated Extension States:
**AL HR LT LV MK**

(30) Priority: **31.07.2003 US 631100**

(71) Applicant: **Delphi Technologies, Inc.
Troy, MI 48007 (US)**

(72) Inventors:
- **Partin, Dale L.**
 **Ray Township, MI 48096 (US)**
- **Prieto, Raymundo**
 **Kokomo, IN 46901 (US)**
- **Sultan, Michel F.**
 **Troy, MI48098 (US)**
- **Wagner, Steve J.**
 **Greentown, IN 46936 (US)**
- **Thrush, Christopher M.**
 **Shelby Township, MI 48316 (US)**

(74) Representative: **Denton, Michael John et al
Delphi European Headquarters,
64 avenue de la Plaine de France,
Paris Nord II,
BP 60059,
Tremblay-en-France
95972 Roissy Charles de Gaulle Cédex (FR)**

(54) **Method for monitoring respiration and heart rate using a fluid -filled bladder**

(57) Respiration and heart rate are monitored using a fluid-filled bladder (12), where the bladder pressure is measured (16) and processed to identify minute pressure variations corresponding to the respiration and heart rate of a subject that is directly or indirectly exerting a load on the bladder (12). The respiration rate is identified by band-pass filtering the measured pressure to isolate or extract a pressure component in range of 0.15-0.5Hz (36), and the heart rate is identified by band-pass filtering the measured pressure to isolate or extract a pressure component in the range of 2-7 Hz (24). The extracted pressure components are preferably converted to a digital format and tabulated for comparison with specified thresholds to identify abnormalities and/or anomalies (30).

## Description

Technical Field

The present invention is related to respiration and heart rate monitoring, and more particularly to a method for monitoring respiration and heart rate based on pressure variation in a fluid-filled bladder disposed in a seat or mattress.

Background of the Invention

Respiration rate, heart rate and their variability are frequently measured as a means of diagnosing and/or analyzing a patient's medical state of health. Such measurements are also indicative of stress level, and a patient is sometimes "wired" to continuously monitor respiration and heart rate during routine or specified situations. It has also been proposed to monitor the respiration and heart rate and the variability of heart rate of the driver of a motor vehicle for purposes of determining the driver's awareness level. Blood pressure and its variability and respiration volume and its variability are also important for analyzing a patient's state of health. Changes in any of these physiological parameters with time may be indicative of a driver's level of awareness, stress, workload or fatigue.

In the case of a vehicle seat, coarse parameters such as occupant weight and presence can be monitored by placing a fluid-filled bladder in or beneath the seat cushion, and measuring the fluid pressure in the bladder; see for example, the U.S. Patent Nos. 5,987,370 and 6,246,936 to Murphy et al., and the U.S. Patent Nos. 6,101,436 and 6,490,936 to Fortune et al., all of which are assigned to Delphi Technologies, Inc. The average fluid pressure in the bladder is proportional to the occupant weight, and variation in the measured pressure as the vehicle is driven can be used to indicate that the occupant is a normally seated child or adult, as opposed to a tightly cinched child seat or infant seat.

Although the bladder-based occupant weight/characterization sensing apparatus is advantageous in that it offers passive and non-intrusive sensing, the information deduced from the pressure measurement has been relatively limited. Accordingly, what is needed is a sensing technique that is passive and non-intrusive in the sense of the seat bladder apparatus, but that is capable of monitoring occupant respiration and heart rate.

Summary of the Invention

The present invention is directed to an improved method for monitoring quasi-periodic physiological functions such as respiration and heart rate using a fluid-filled bladder disposed in a seat or mattress, wherein the bladder pressure is measured and processed to identify minute pressure variations corresponding to the respiration and heart rate of a person that is directly or indirectly exerting a load on the bladder. The respiration rate is identified by band-pass filtering the measured pressure to isolate or extract a pressure component which may be in the range of 0.15-0.5Hz, and the heart rate is identified by band-pass filtering the measured pressure to isolate or extract a pressure component which may be in the range of 2-7 Hz. The extracted pressure components are preferably converted to a digital format, processed and tabulated for comparison with specified thresholds to identify abnormalities and/or anomalies. While the above physiological functions can be characterized by a rate, frequency or periodicity, the characteristics also vary with time, and their variability can be separately measured. This is also true of the amplitudes of the respective pressure components that are related to differential blood pressure and respiration volume. For this reason, the physiological functions are considered to be quasi-periodic.

Brief Description of the Drawings

Figure 1 is a diagram of a motor vehicle seat including a fluid-filled seat bladder and processing circuitry in accordance with this invention.
Figure 2 graphically depicts the AC content of a measured pressure of the fluid in the seat bladder of Figure 1, and two isolated components of such pressure.
Figure 3 is a graph depicting a processed version of one of the components signals depicted in Figure 2.
Figure 4 depicts a representative sampling of heartbeat frequency according to this invention.

Description of the Preferred Embodiment

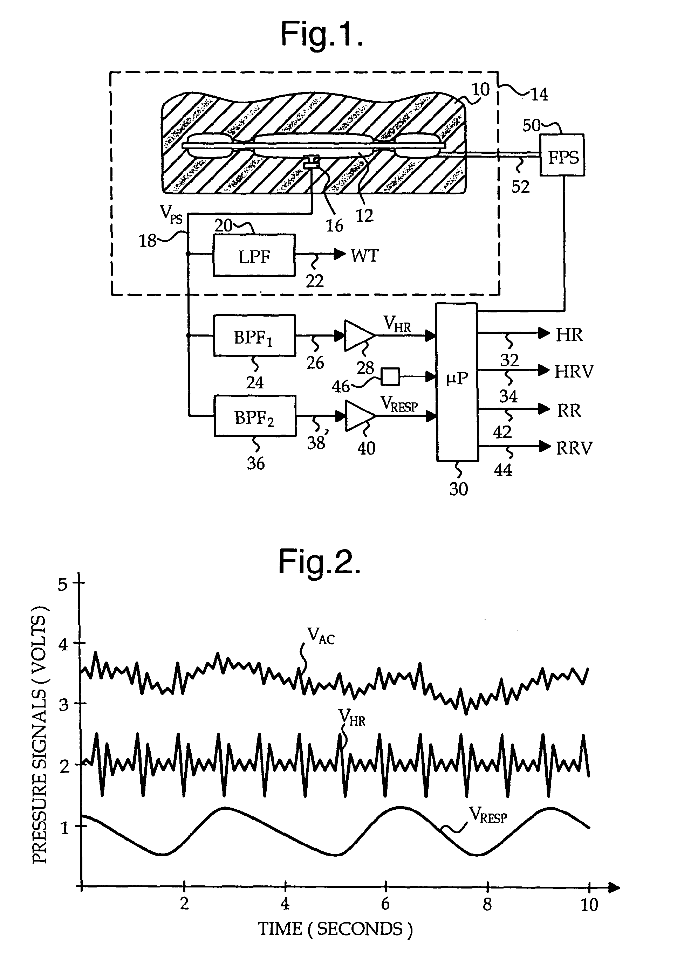

Referring to Figure 1, the present invention is illustrated in the context of a motor vehicle seat cushion 10 equipped with a fluid-filled seat bladder 12. However, it will be recognized that the invention is not limited to motor vehicle applications, and is applicable to other environments and contexts, such as in a wheelchair, bed, crib, etc. Also, the bladder 12 may be installed under the seat cushion 10 instead of in it, as disclosed for example, in the aforementioned U.S. Patent No. 6,490,936 to Fortune et al., incorporated by reference herein. The components within the region designated by the reference numeral 14 represent the various elements typically present in a vehicular occupant weight sensing system of the type disclosed in the aforementioned patents. In addition to the bladder 12, such elements include a pressure sensor 16 for producing a pressure signal ($V_{PS}$) on line 18, and a low-pass filter (LPF) 20 for producing an occupant weight signal (WT) on line 22. The pressure sensor 16 detects the pressure of the bladder fluid at a point at or near its center-of-mass. The low-pass filter 20 is designed to remove perturbations of the pressure signal $V_{PS}$ associated with occupant movement and so forth so that the weight signal WT is essentially the DC component of the pressure signal $V_{PS}$.

Fundamentally, the present invention recognizes that certain perturbations of the pressure signal $V_{PS}$ are associated with quasi-periodic physiological functions of the occupant such as breathing and heart rate, and that such perturbations can be isolated to provide respiration and heart rate information about the occupant. Depending on the mechanical construction of the seat (or mattress, for example), the fundamental heart rate frequency as well as its harmonics will be transmitted to the bladder 12, the fundamental frequency being in the range of about 0.6 Hz to about 3 Hz. Frequency components above about 10Hz can usually be ignored. Infants and children tend to have heart and respiration rates that are higher than those of adults, and this may require an increase in the monitored frequency ranges. For some purposes, it is desired to determine the pulse-to-pulse interval rather than the heart rate or heart beat frequency.

If desired, the system of Figure 1 may be modified to optimize one or more signal components. For example, the system may include multiple bladders for optimizing physiological information from different locations or to process the various output signals differentially in order to reduce the effects from body movement, vehicle vibration or noise. A single bladder with two or more pressure sensors can also be used for similar purposes since the pressure in a bladder may have spatially local transients. Also, the effects of vehicle vibration or other environmental disturbances can be attenuated and/or compensated for by sensing the presence of such vibration or disturbances with an accelerometer 46, for example. Additionally, the heart and respiration rate components may be optimized by adjusting the base inflation pressure of the bladder 12; to this end, the embodiment of Figure 1 illustrates a fluid pumping system (FPS) 50 coupled to the bladder 12 by a flexible conduit 52. Depending upon the system implementation, measurement of the heart rate and respiration rate components may be optimized with a higher inflation pressure. However, higher inflation pressures may cause the bladder 12 to be too firm for patient comfort. Thus, the optimum inflation pressure will typically involve a trade-off between signal level and patient comfort.

In general, the perturbations associated with respiration and heart rate can be detected by band-pass filtering the pressure signal $V_{PS}$ to identify the signal components in the frequency range of about 0.1Hz - 30Hz or 0.3Hz - 30Hz. The resulting signal $V_{AC}$ is depicted in Figure 2, with a DC offset voltage of approximately 3.5 volts. The relatively low frequency undulation of the waveform is due to the occupant's respiration, whereas the higher frequency undulation is due to the occupant's heart beat.

Referring to Figure 1, the reference numeral 24 designates a band-pass filter $BPF_1$ for specifically identifying the frequency components of the pressure signal $V_{PS}$ associated with the occupant's heartbeat, and the reference numeral 36 designates a band-pass filter $BPF_2$ for specifically identifying the frequency components of the pressure signal $V_{PS}$ associated with the occupant's respiration. In the illustrated embodiment, the band-pass filter $BPF_1$ is configured to pass components of the pressure signal $V_{PS}$ in the frequency range of 2Hz to 7Hz, producing an output signal such as the trace $V_{HR}$ in Figure 2; the band-pass filter $BPF_2$ is configured to pass components of the pressure signal $V_{PS}$ in the frequency range of 0.15Hz to 0.5Hz, producing an output signal such as the trace $V_{RESP}$ in Figure 2. As with the trace $V_{AC}$, the traces $V_{HR}$ and $V_{RESP}$ are illustrated with DC offsets so that the traces can be viewed separately. The output of band-pass filter 24 on line 26 is amplified by the amplifier 28 and supplied to an A/D input port of the microprocessor 30. Similarly, the output of band-pass filter 36 on line 38 is amplified by the amplifier 40 and supplied to an A/D input port of the microprocessor 30. The microprocessor 30, which could alternatively be implemented with a digital signal processor, functions to process the input signals to form output signals on lines 32, 34, 42 and 44 representative of the occupant's heart rate (HR), heart rate variability (HRV), respiration rate (RR) and respiration rate variability (RRV). Of course, the microprocessor 30 could also be programmed to compare the depicted outputs with threshold values indicative of normal or marginally abnormal values, and to activate an alarm or warning device when abnormalities or anomalies are detected. Also, it may be desirable to detect changes in the values of HR, HRV, RR and RRV that occur over time for a given individual for purposes of detecting the onset of drowsiness or over-stressing. The same is true of the differential blood pressure (that is, the difference between the systolic and diastolic blood pressures) and respiration volume. The amplitude of the pressure variations due to the heart pulses are also approximately linearly related to the differential blood pressure. The amplitude of the pressure variations due to respiration are approximately linearly related to the volume of breath exchanged. These physiological parameters and their variability with time can also be monitored as an indication of stress, awareness level, etc.

The signal processing performed by microprocessor 30 to extract the HR and HRV outputs can include local normalization and exponentiation. The signal $V_{HR}$ may be normalized locally according to the following scheme:

$$V_{NORM}(t) \ = \ \frac{V_{HR}(t) - V_{MIN}\left(t - \frac{T_w}{2} \leq t \leq t + \frac{T_w}{2}\right)}{V_{MAX}\left(t - \frac{T_w}{2} \leq t \leq t + \frac{T_w}{2}\right) - V_{MIN}\left(t - \frac{T_w}{2} \leq t \leq t + \frac{T_w}{2}\right)} \quad (1)$$

where $V_{MIN}$ is the minimum $V_{HR}$ signal that occurs in the time interval

$$\left(t - \frac{T_w}{2} \leq t \leq t + \frac{T_w}{2}\right)$$

and $V_{MAX}$ is the maximum $V_{HR}$ signal that occurs in the same time interval. The time window $T_w$ is selected to be slightly lower than the HR repetition interval, and may be adaptively adjusted if desired. By way of example, $T_w$ may be fixed at 0.8 seconds. In an adaptive configuration, $T_w$ may be reset to 80%-90% of the previously determined pulse-to-pulse duration to ensure that any close-by structured peaks are not confused as heart pulses, while ensuring that the previous or next heart pulses are still counted as heart pulses. Normalizing the $V_{HR}$ signal allows the signal peaks to be easily identified since the peaks all assume a value of unity while the remainder of the normalized waveform has values between zero and unity. The normalization can be further enhanced by raising the locally normalized signal to a power $N$:

$$V_{NORM\text{-}EXP}(t) = (V_{NORM}(t))^N \quad (2)$$

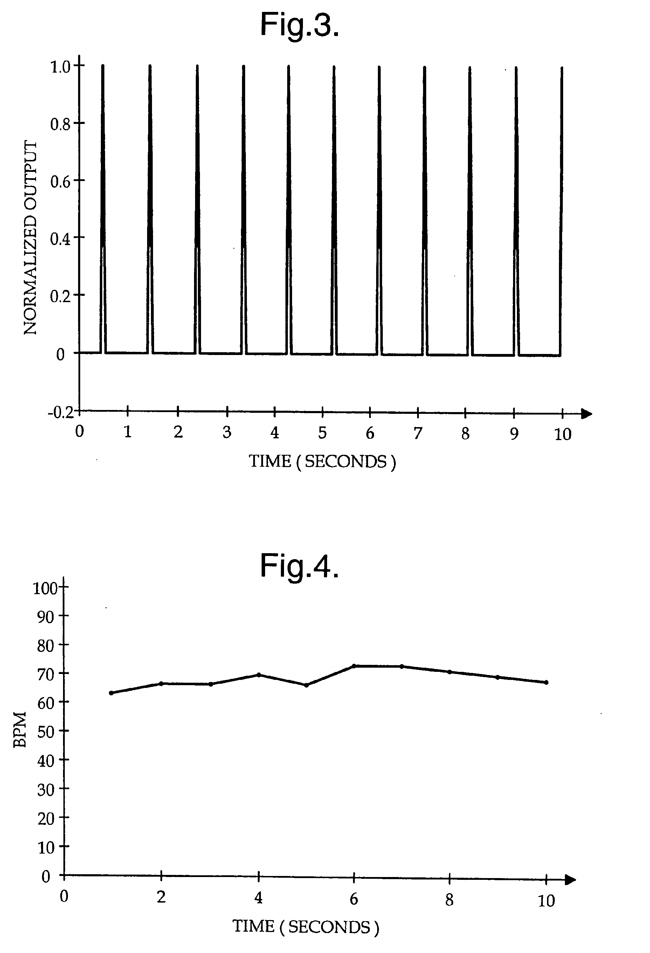

where N = 15, for example. The result of such exponentiation is depicted in Figure 3. Referring to Figure 3, it will be seen that only heart rate pulses remain in the $V_{NORM\_EXP}$ signal, and that other perturbations are greatly attenuated. As illustrated in Figure 4, the heart rate HR in beats per minute (BPM) can be easily obtained from either the normalized or normalized-exponentiated waveforms, where HR = 60/Tp, with Tp representing the pulse-to-pulse interval. Heart rate variability HRV may be determined by calculating the variance of Tp, for example. Alternatively, the microprocessor 30 may perform additional signal processing in the frequency domain (FFT, power spectrum, harmonic spacing, etc.) or the time domain (correlation, adaptive digital filtering, amplification, compensation from other inputs, etc.). In a similar manner, the respiration rate RR may be determined by one of the techniques used for heart rate. If the local normalization technique is used, a larger window size is needed to account for the lower respiration rate. Other schemes such as zero crossing detection could also be used. In some cases, the respiration rate variability (RRV) as well as respiration rate (RR) is of interest; this may be detected in a manner similar to the detection of heart rate variability (HRV).

In summary, the present invention provides a passive, non-intrusive and inexpensive method for monitoring physiological functions such as respiration and heart rate. While described in reference to a human occupant of a vehicle seat, it will be understood that the method equally applies to subjects other environments, and even to non-human subjects that exhibit quasi-periodic physiological functions such as respiration and heart rate.

On an implementation level, it will be recognized that the pressure signal $V_{PS}$ may be transmitted to the detection circuitry by a wireless communication system, if desired, and that the amplifier and filter elements depicted in Figure 1 may be reversed, or the microprocessor 30 replaced with a digital signal processor, as mentioned above. Further, additional band-pass filters may be utilized to detect and monitor body movements, and to detect body movements that are characteristic of choking, convulsions, seizures, coughing, childbirth contractions, etc. The pressure signal $V_{PS}$ and/or the processed HR, HRV, RR or RRV signals may be transmitted wirelessly to a remote site after a vehicle collision in order to assess a medical condition, including whether the occupant is alive or present. In such a case, the presence of the occupant may be determined from the occupant weight signal WT. Auxiliary signals may be included to assist in determining if the vehicle has been over-turned or if the occupant's seat belt is still fastened. Also, the invention may be applied to various types of vehicles, such as aircraft, and to non-automotive uses such as wheelchairs, bed, cribs and so on. As with automotive applications, a wireless communication could be made to alert medical personnel of an accident condition and assess the medical condition of the subject. Additionally, the invention may involve communications to the subject/patient or another person based on the processed signals, such as a communication that the subject/patient is not moving frequency enough for good health. Moreover, the measured heart and respiration rates can be used as indicators of stress or nervous activity level, from which various conclusions can be inferred; for example, high respiration and heart rate in the case of an aircraft passenger may be used as an indication of extreme nervousness or possible criminal intent. In this regard, it should be understood that methods incorporating these and other modifications may fall within the scope of this invention, which is defined by the appended claims.

**Claims**

1. A method of monitoring a quasi-periodic physiological function of a subject, comprising the steps of:

   locating a fluid-filled bladder (12) in a supportive load-bearing relationship with respect to the subject;
   measuring a fluid pressure in the bladder (16);
   isolating a perturbation of the measured pressure due to said periodic physiological process (24, 36); and
   identifying and monitoring at least a frequency or period of said perturbation (30).

2. The method of Claim 1, wherein the quasi-periodic physiological function is a heart rate of said subject, and the step of isolating a perturbation of the measured pressure due to said heart rate includes band-pass filtering perturbations of the measured pressure in the range of about 0.6Hz to 10Hz (24).

3. The method of Claim 2, wherein the band-pass filtering is in the range of about 2Hz to 7Hz (24).

4. The method of Claim 2, including the step of:

   determining a variability of the isolated perturbation to determine heart rate variability (30, 34).

5. The method of Claim 2, including the step of:

   determining an amplitude of said perturbation as an indication of the subject's differential blood pressure (30).

6. The method of Claim 5, including the step of:

   measuring a variability of the determined amplitude with respect to time (30).

7. The method of Claim 5, including the step of:

   using said amplitude as an indication of the subject's health, alertness, awareness or impairment (30).

8. The method of Claim 1, wherein the quasi-periodic physiological function is a respiration rate of said subject, and the step of isolating a perturbation of the measured pressure due to said respiration rate includes band-pass filtering perturbations of the measured pressure in the range of about 0.15Hz to 0.5Hz (36).

9. The method of Claim 8, including the step of:

   determining a variability of the isolated perturbation to determine respiration rate variability (30, 44).

10. The method of Claim 8, including the step of:

    determining an amplitude of the isolated perturbation as an indication of the subject's respiration volume (30).

11. The method of Claim 10, including the step of:

    measuring a variability of the determined amplitude with respect to time (30).

**12.** The method of Claim 10, including the step of:

using said amplitude as an indication of the subject's health, alertness, awareness or impairment (30).

**13.** The method of Claim 1, including the step of:

adjusting an inflation level of said bladder (12) to optimize the measured pressure and comfort of the subject (30, 50, 52).

**14.** The method of Claim 1, wherein there are two or more fluid-filled bladders (12), and the measured pressure is a differential pressure between the bladders (12).

**15.** The method of Claim 1, including the steps of:

independently measuring environmental disturbances that affect the measured pressure (46); and compensating the measured pressure for such independently measured environmental disturbances (30).

**16.** The method of Claim 1, including the step of:

measuring a variability of the isolated perturbation with respect to time (30).

**17.** The method of Claim 1, including the step of:

using the monitored frequency or period of said perturbation as an indication of the subject's health, alertness, awareness or impairment (30).

**18.** The method of Claim 1, including the step of:

using said frequency or period of said perturbation as an indication of possible criminal intent of the subject (30).

**19.** The method of Claim 1, wherein the subject is disposed in a vehicle, and the method includes the step of:

using said frequency or period of said perturbation to assess a medical condition of the subject after a collision of the vehicle, including whether the subject is alive or present (30).

**20.** The method of Claim 19, including the step of:

confirming the presence of the subject by determining a weight of the subject from a DC pressure (20, 22) in said bladder (12).

**21.** The method of Claim 19, including the step of:

determining that said vehicle has overturned or that said subject is still wearing a seat belt (30).

**22.** The method of Claim 19, including the step of:

automatically communicating said medical condition (30).

**23.** A method of monitoring a non-periodic physiological disorder of a subject, comprising the steps of:

locating a fluid-filled bladder (12) in a supportive load-bearing relationship with respect to the subject;
measuring a fluid pressure in the bladder (16);
monitoring abnormally large variations in the measured pressure (30); and
using said abnormally large variations to detect choking, convulsions, seizures, coughing, maternal contractions or frequency of movement of said subject (30).

**24.** The method of Claim 23, including the steps of:

independently measuring environmental disturbances that affect the measured pressure (46); and compensating the measured pressure for such independently measured environmental disturbances (30).

**25.** The method of Claim 23, including the step of:

using said abnormally large variations as an indication of the subject's health, alertness, awareness or impairment (30).

**26.** The method of Claim 23, including the step of:

communicating to the subject or another person if the subject is not moving enough for good health (30).

**27.** The method of Claim 23, including the step of:

using said abnormally large variations as an indication of possible criminal intent of the subject (30).

**28.** The method of Claim 23, wherein the subject is disposed in a vehicle, and the method includes the step of:

using said abnormally large variations to assess a medical condition of the subject after a collision of the vehicle, including whether the subject is alive or present (30).

**29.** The method of Claim 28, including the step of:

confirming the presence of the subject by determining a weight of the subject from a DC pressure (20, 22) in said bladder (12).

**30.** The method of Claim 28, including the step of:

determining that said vehicle has overturned or that said subject is still wearing a seat belt (30).

**31.** The method of Claim 28, including the step of:

automatically communicating said medical condition (30).

# Fig.1.

# Fig.2.

# Fig.3.

# Fig.4.

**European Patent Office**

**EUROPEAN SEARCH REPORT**

Application Number

EP 04 07 7060

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 1 247 488 A (M I LAB CORP) 9 October 2002 (2002-10-09) <br><br> * paragraphs [0009], [0010], [0018] - [0024], [0032], [0036], [0040], [0044], [0060] * <br> * claims 1-3 * | 1,14,16, 17,23, 25,26 | A61B5/08 A61B5/0205 A61B5/11 |
| Y <br> A |  | 2-4,8,9 <br> 5-7, 10-13, 15, 18-22, 24,27-31 |  |
| Y | US 4 686 999 A (REUSS JAMES L  ET AL) 18 August 1987 (1987-08-18) <br> * column 5, lines 56-61 * <br> * column 6, lines 7-18 * <br> * column 6, lines 30-47 * <br> * column 9, line 58 - column 10, line 3 * | 2-4,8,9 |  |
| X | CA 2 422 818 A (M I LAB CORP) 22 July 2003 (2003-07-22) <br> * page 6 - page 10 * | 1,16,17 | TECHNICAL FIELDS SEARCHED (Int.Cl.7) <br><br> A61B |
| A |  | 19-22, 28-31 |  |
| X | PATENT ABSTRACTS OF JAPAN vol. 0176, no. 22 (C-1130), 17 November 1993 (1993-11-17) & JP 5 192315 A (MITSUBISHI HEAVY IND LTD; others: 01), 3 August 1993 (1993-08-03) * abstract * | 1,14 |  |

The present search report has been drawn up for all claims

| Place of search | Date of completion of the search | Examiner |
|---|---|---|
| Munich | 4 October 2004 | Rivera Pons, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 04 07 7060

This annex lists the patent family members relating to the patent documents cited in the above-mentioned European search report.
The members are as contained in the European Patent Office EDP file on
The European Patent Office is in no way liable for these particulars which are merely given for the purpose of information.

04-10-2004

| Patent document cited in search report | | Publication date | Patent family member(s) | | Publication date |
|---|---|---|---|---|---|
| EP 1247488 | A | 09-10-2002 | JP | 3242631 B2 | 25-12-2001 |
| | | | JP | 2001145605 A | 29-05-2001 |
| | | | JP | 3419732 B2 | 23-06-2003 |
| | | | JP | 2001276019 A | 09-10-2001 |
| | | | AU | 6866300 A | 04-06-2001 |
| | | | CA | 2377903 A1 | 31-05-2001 |
| | | | EP | 1247488 A1 | 09-10-2002 |
| | | | NO | 20016214 A | 05-02-2002 |
| | | | CN | 1373643 T | 09-10-2002 |
| | | | WO | 0137731 A1 | 31-05-2001 |
| US 4686999 | A | 18-08-1987 | EP | 0218690 A1 | 22-04-1987 |
| | | | WO | 8605965 A1 | 23-10-1986 |
| CA 2422818 | A | 22-07-2003 | WO | 03061476 A1 | 31-07-2003 |
| | | | JP | 3495982 B2 | 09-02-2004 |
| | | | JP | 2002187450 A | 02-07-2002 |
| | | | BR | 0206390 A | 06-07-2004 |
| | | | CA | 2422818 A1 | 22-07-2003 |
| | | | US | 2004061615 A1 | 01-04-2004 |
| JP 5192315 | A | 03-08-1993 | NONE | | |

EPO FORM P0459

For more details about this annex : see Official Journal of the European Patent Office, No. 12/82